# FASCICULE DE BREVET EUROPEEN

(11) **EP 2 958 953 B1**
(45) Date de publication et mention de la délivrance du brevet: **18.07.2018**
(21) Numéro de dépôt: 14713178.3
(22) Date de dépôt: 25.02.2014
(51) Int. Cl.: C08G 69/34, C08G 69/48, C08G 83/00, C07D 233/36

(54) **MATÉRIAUX SUPRAMOLÉCULAIRES À BASE D'OLIGO-AMIDES**
SUPRAMOLEKULARE STOFFE AUS OLIGOAMIDEN
SUPRAMOLECULAR MATERIALS MADE OF OLIGOAMIDES

(30) Priorité: 25.02.2013 FR 1351643
(43) Date de publication de la demande: 30.12.2015
(73) Titulaire: Arkema France, 92700 Colombes (FR); Centre National de la Recherche Scientifique (C.N.R.S.), 75794 Paris Cedex 16 (FR); Ecole Supérieure de Physique et de Chimie Industrielles de la Ville de Paris, 75005 Paris (FR)
(72) Inventeur: LEIBLER, Ludwik, F-75006 Paris (FR); TOURNILHAC, François, F-75012 Paris (FR); CAPELOT, Mathieu, 27470 SERQUIGNY (FR); AGNAOU, Réda, F-93110 Rosny sous Bois (FR); PINEAU, Quentin, F-27000 Evreux (FR)
(74) Mandataire: Bonnel, Claudine
(86) Numéro de dépôt international: PCT/FR2014/050398
(87) Numéro de publication internationale: WO 2014/128426

(56) Documents cités:
- WO-A1-2006/016041
- WO-A1-2006/087475
- WO-A1-2010/031965
- WO-A1-2012/052673
- WO-A1-2012/156610
- WO-A2-2009/141558
- WO-A2-2011/015773
- "Polyamide" In: "Kunststoff-Handbuch", 1966, Carl Hanser Verlag, München, XP002716476, vol. VI, pages 387-389, le document en entier
- MONTARNAL D ET AL: "Versatile one-pot synthesis of supramolecular plastics and self-healing rubbers", JOURNAL OF THE AMERICAN CHEMICAL SOCIETY, vol. 131, no. 23, 17 juin 2009 (2009-06-17), pages 7966-7967, XP002716512, AMERICAN CHEMICAL SOCIETY USA DOI: 10.1021/JA903080C
- CARTTON E ASH SHELL CHEMICAL COMPANY HOUSTON TX ET AL: "Multilayer aliphatic polyketone/polyamide polymer structures (Carlton E. Ash, Dixie G. Waters, Richard L. Danforth, Shell Chemical Company; Todd F. Volyn, Shell Oil Company)", RESEARCH DISCLOSURE, MASON PUBLICATIONS, HAMPSHIRE, GB, vol. 406, no. 21, 1 février 1998 (1998-02-01), XP007122370, ISSN: 0374-4353

## Description

### DOMAINE TECHNIQUE

La présente invention concerne un procédé de préparation de matériaux supramoléculaires à base d'oligo-amides linéaires ou ramifiés terminés à chacune de leurs extrémités par un groupe associatif comprenant un hétérocycle azoté porté par une séquence particulière.

### ARRIERE-PLAN DE L'INVENTION

Les matériaux dits supramoléculaires sont des matériaux constitués de molécules associées par des liaisons non covalentes, telles que des liaisons hydrogène, ioniques et/ou hydrophobes. Un avantage de ces matériaux est que ces liaisons physiques sont réversibles, notamment sous l'influence de la température ou par l'action d'un solvant sélectif.

Certains d'entre eux possèdent en outre des propriétés élastomères. Contrairement aux élastomères classiques, ces matériaux ont l'avantage de pouvoir se fluidifier au-dessus d'une certaine température, ce qui facilite leur mise en oeuvre, notamment le bon remplissage des moules, ainsi que leur recyclage. Bien qu'ils ne soient pas constitués de polymères réticulés mais de petites molécules, ces matériaux sont, comme les élastomères, capables de présenter une stabilité dimensionnelle sur des temps très longs et de recouvrer leur forme initiale après de grandes déformations. Ils peuvent être utilisés pour fabriquer des isolants thermiques ou acoustiques, des câbles, des gaines, des semelles de chaussures, des emballages, des colliers de serrage élastiques, des tubes à vide, ou encore des tubes de transport de fluides.

Des matériaux supramoléculaires ont déjà été décrits par la Demanderesse, notamment dans les documents WO 03/059964, WO 2006/016041, WO 2006/087475, WO 2008/029065, WO 2009/141558, WO 2012/156610 et WO 2012/052673 et dans la publication de D. Montarnal et al., Journal of the American Chemical Society, Vol. 131, No. 23, pp. 7966-7967 (2009).

Parmi les documents précités, le document WO 2006/016041 décrit un polymère modifié par greffage d'AEIO (ou UDETA) ou d'un dérivé d'UDETA. Dans le cas où le polymère est un polyamide, en particulier un copolyamide du type Platamid®, de l'UDETA est greffée sur ses extrémités acides, (qui sont de différents types dans le cas du Platamid®). Le produit de cette réaction est donc constitué d'un mélange de composés présentant des fins de chaîne différentes. Ce greffage a en outre pour effet d'augmenter la viscosité du polyamide, jusqu'à atteindre une valeur de G" correspondant à une viscosité de 17 Pa.s. Une viscosité plus élevée encore (supérieure à 7000 Pa.s) est observée pour les autres polymères greffés exemplifiés dans ce document. Les polymères décrits dans WO 2009/141558 ont également une viscosité trop élevée (supérieure à 225 Pa.s dans l'Exemple 1).

Le document WO 2011/015773 décrit des polycondensats capables de former une structure supramoléculaire. Dans le cas où le polycondensat est un polyamide, un composé comprenant au moins un groupement associatif, tel que l'UDETA, peut être mis en présence des monomères pendant la réaction de polycondensation. L'incorporation de ces polycondensats dans un bitume permet d'améliorer les propriétés mécaniques du bitume.

En outre, le document WO 2010/031965 décrit un procédé pour compatibiliser deux polymères par greffage d'UDETA. L'exemple 1 divulgue un matériau formé par réaction d'acide adipique sur un polyamide de type PA-11, suivie de la fonctionnalisation du produit obtenu à l'aide d'UDETA. Là encore, ce composé présente une viscosité trop élevée.

Bien que ces matériaux, et plus particulièrement ceux présentant un caractère élastomérique, offrent des propriétés très satisfaisantes à température ambiante, il serait souhaitable de disposer de matériaux présentant de bonnes propriétés mécaniques à l'état solide, approchant celles des matériaux thermoplastiques, tels qu'une contrainte et un allongement à la rupture élevés, et plus particulièrement une contrainte à la rupture supérieure à 3 MPa, de préférence à 4 MPa, voire à 10 MPa, sur une large plage de températures, tout en conservant une faible viscosité à l'état fondu, similaire à celle des huiles (typiquement inférieure à 10 Pa.s voire à 1 Pa.s). Il serait ainsi possible d'améliorer l'aptitude à la mise en oeuvre de ces matériaux, notamment leur capacité de mouillage de fibres permettant de les utiliser dans la fabrication de composites fibreux, ainsi que leur capacité de recyclage et leur durabilité.

Or, les tentatives visant à réduire la viscosité des matériaux supramoléculaires connus, en diminuant leur poids moléculaire, ont systématiquement conduit à des produits présentant une contrainte à la rupture inférieure à 4 MPa et quelques pourcent seulement d'allongement à la rupture. Les inventeurs ont découvert que les deux exigences précitées, pourtant difficilement conciliables, pouvaient être satisfaites par un matériau supramoléculaire qui se différencie de ceux connus en ce qu'il est semi-cristallin, avec un point de fusion supérieur ou égal à 120°C et de préférence supérieur ou égal à 140°C. De manière tout-à-fait surprenante, ce matériau comporte un groupe associatif de type UDETA réputé augmenter la viscosité des polymères sur lesquels il est greffé. Sans vouloir être liés par cette théorie, il semble que les cristallites présents en-deçà de la température de fusion du matériau jouent le rôle de points de réticulation et permettent de conférer à celui-ci les propriétés thermiques et mécaniques recherchées. Ce matériau peut être obtenu suivant un procédé aisément industrialisable, par polycondensation d'un diacide, d'une diamine et d'un composé modificateur porteur d'un groupe associatif situé à l'extrémité d'une séquence particulière, ces réactifs étant utilisés dans des rapports stoechiométriques donnés. Il a été mis en évidence que les groupes associatifs terminaux, à condition d'être greffés *via* cette séquence particulière, confèrent au matériau le point de fusion et la cristallinité requis, tandis que la chaîne oligo-amide centrale présente une flexibilité suffisante pour contribuer à l'obtention des propriétés mécaniques recherchées. Dans une forme préférée de l'invention, cette dernière est plus particulièrement constituée d'une alternance de blocs rigides (formés par les motifs diacide) et souples (formés par les motifs diamine). En outre, le composé modificateur permet de réduire la taille des chaînes de molécules constituant le matériau et de lui conférer ainsi une faible viscosité à l'état fondu.

### RESUME DE L'INVENTION

L'invention a pour objet un procédé de préparation d'un matériau comprenant des oligo-amides X.Y linéaires ou ramifiés, terminés à plus de 90% en nombre de leurs extrémités par un même groupe -M-L₂-CO-L₁-A dans lequel M est choisi parmi les groupes CO, NH et O, ledit procédé comprenant une étape de polycondensation :
(a) d'au moins un acide dicarboxylique,
(b) d'au moins une diamine, et
(c) d'au moins un composé modificateur de formule A-L₁-CO-L₂-W préalablement synthétisé et isolé, où : A est un groupe associatif comprenant un hétérocycle azoté ; L₁ est une liaison chimique ou un bras espaceur constitué d'une chaîne hydrocarbonée saturée ou insaturée, cyclique ou non, éventuellement interrompue par un ou plusieurs atomes d'oxygène et/ou d'azote ; L₂ est une chaîne hydrocarbonée saturée ou insaturée, cyclique ou non, renfermant au moins 4 atomes de carbone, éventuellement interrompue par un ou plusieurs groupes oxo et éventuellement substituée par un ou plusieurs groupes -OH et/ou un ou plusieurs atomes de chlore ; W est un groupe réactif capable de réagir :
   - soit avec les fonctions amines de la diamine, le rapport molaire des fonctions acides du diacide aux fonctions réactives du composé modificateur allant dans ce cas de 1 à 8, étant entendu que le nombre de moles de fonctions amines de la diamine est égal à la somme du nombre de moles des fonctions acides et réactives précitées,
   - soit avec les fonctions acides du diacide, le rapport molaire des fonctions amines de la diamine aux fonctions réactives du composé modificateur allant dans ce cas de 1 à 8, étant entendu que le nombre de moles de fonctions acides du diacide est égal à la somme du nombre de moles des fonctions amines et réactives précitées.

La présente invention décrit les utilisations de ce matériau comme additif dans les bitumes, comme matrice de matériaux composites, comme adhésif thermofusible ou comme additif dans des adhésifs thermofusibles.

Le composé de formule : 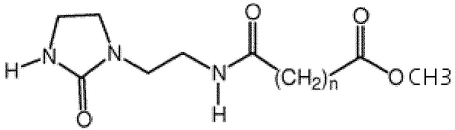 où n va de 4 à 14, est préféré comme composé modificateur dans le procédé selon l'invention.

### DESCRIPTION DETAILLEE

A titre de préambule, on notera que l'expression "compris entre" doit être interprétée, dans la présente description, comme incluant les bornes citées.

Comme indiqué précédemment, le matériau supramoléculaire préparé selon l'invention comprend des oligo-amides X.Y linéaires ou ramifiés terminés à plus de 90% en nombre de leurs extrémités par un même motif particulier, et préférentiellement à 100%.

Par le terme oligo-amides, on désigne des polycondensats ayant une faible masse moléculaire moyenne en nombre, Mn. La valeur de Mn peut être prédite en fonction des rapports molaires des réactifs intervenant dans la polycondensation et du degré d'avancement de la réaction, en utilisant les formules classiques, connues de l'homme du métier. On choisira les rapports molaires de telle façon que la Mn prévue par la formule de Stockmayer, W.H. (Journal of Polymer Science 1952, 9, 67-71), et en supposant une conversion totale, soit inférieure à 10000 g/mol et de préférence inférieure à 4500g/mol.

Par "oligo-amides X.Y", on entend des homopolyamides ou copolyamides de faible masse obtenus à partir d'au moins un diacide et d'au moins une diamine, par opposition aux oligo-amides contenant des motifs obtenus par condensation d'amino-acides.

Le terme "oligo-amide linéaire" désigne des oligo-amides ne comportant dans la chaîne que des unités diacides et diamines, le terme "oligo-amides ramifiées" désigne des oligo-amides comportant dans la chaîne une ou plusieurs unités polyacide ou polyamine de fonctionnalité supérieure à 2.

En outre, par l'expression " plus de 90% en nombre...", on entend que moins de 10% en nombre des extrémités des molécules constituant le matériau peuvent résulter éventuellement d'une conversion incomplète de la réaction ou peuvent provenir d'espèces monofonctionnelles présentes dans les monomères utilisés. Il s'agit plus particulièrement de fonctionnalités acide, amine, ou alkyle, observables par les techniques analytiques classiques, telles que la potentiométrie, la RMN du proton ou la spectroscopie infrarouge.

Selon un premier mode de réalisation de l'invention, le matériau obtenu selon le procédé de l'invention comprend des oligomères répondant à la formule (Ia) ci-dessous :

X-NH-Ra-NH-[CO-Rb-CO-NH-Ra-NH-]ₐ-X **(Ia)**

où :
Ra désigne une chaîne hydrocarbonée saturée ou insaturée, éventuellement interrompue par un ou plusieurs atomes d'oxygène et/ou d'azote,
Rb désigne une chaîne hydrocarbonée saturée ou insaturée,
a désigne le nombre moyen de motifs par chaîne et est supérieur à 0 et inférieur ou égal à 20, préférentiellement inférieur ou égal à 9, et encore plus préférentiellement compris entre 1 et 3,
X désigne un groupement A-L₁-CO-L₂-CO-
   où :
   A est un groupe associatif comprenant un hétérocycle azoté,
   L₁ est une liaison chimique ou un bras espaceur constitué d'une chaîne hydrocarbonée saturée ou insaturée, cyclique ou non, éventuellement interrompue par un ou plusieurs atomes d'azote et/ou d'oxygène,
   L₂ est une chaîne hydrocarbonée saturée ou insaturée, cyclique ou non, renfermant au moins 4 atomes de carbone, éventuellement interrompue par un ou plusieurs groupes oxo et éventuellement substituée par un ou plusieurs groupes -OH et/ou un ou plusieurs atomes de chlore.
   Dans la formule (Ia) ci-dessus, on préfère que le groupe X contienne une séquence L_{1'}-CO-L₂-CO- où L₂ désigne une chaîne alkylène linéaire et L_{1'} désigne une chaîne -NH-CH₂-CH₂-.

Selon un second mode de réalisation, le matériau obtenu selon le procédé de l'invention comprend des oligomères répondant à la formule (Ib) ci-dessous :

X'-CO-Rb-CO-[NH-Ra-NH-CO-Rb-CO-]ₐ-X' **(Ib)**

où :
Ra, Rb et a ont les définitions indiquées ci-dessus,
X' désigne un groupement A-L₁-CO-L₂-M₁-
   où :
   A, L₁ et L₂ ont la définition indiquée ci-dessus,
   M₁ est un atome d'oxygène ou un groupe NH.

Dans les formules (Ia) et (Ib) ci-dessus, on préfère que l'une au moins des chaînes Ra et Rb ne constitue pas une chaîne alkylène linéaire.

Les réactifs utilisés dans le procédé de synthèse de ces matériaux seront maintenant décrits plus en détail.

### Acide dicarboxylique

L'acide dicarboxylique mis en oeuvre dans la première étape du procédé selon l'invention comprend avantageusement de 4 à 100 atomes de carbone. Il peut s'agir d'un acide dicarboxylique linéaire et saturé, renfermant de 4 à 22 atomes de carbone, tel que l'acide succinique, l'acide glutarique, l'acide adipique, l'acide pimélique, l'acide subérique, l'acide azélaïque, l'acide sébacique, l'acide undécanedioïque, l'acide dodécanedioïque, l'acide brassylique, l'acide tétradécanedioïque, l'acide pentadecanedioïque, l'acide thapsique, l'acide octadécanedioïque et leurs mélanges. En variante, l'acide dicarboxylique utilisé dans cette invention peut être un acide dicarboxylique ramifié et saturé, renfermant par exemple de 6 à 10 atomes de carbone, comme l'acide 3,3-diméthyl glutarique.

En variante encore, il peut s'agir d'un diacide aromatique tel que l'acide téréphthalique, l'acide isophthalique, les diacides naphtaléniques et leurs mélanges. Dans une autre variante, le diacide peut être cycloaliphatique. Dans ce dernier cas, il peut comporter les squelettes carbonés suivants : norbornyl méthane, cyclohexylméthane,dicyclohexylméthane,dicyclohexylpropan, di(méthylcyclohexyl), di(méthylcyclohexyl)propane.

On peut également utiliser selon l'invention les dimères d'acides carboxyliques d'origine végétale, constitués de deux monomères d'acides gras identiques ou différents, éventuellement en mélange avec des monomères et/ou des trimères d'acides gras, ces mélanges étant choisis de telle sorte que le polycondensat obtenu reste en-deçà du point de gel chimique. Ces composés d'origine végétale peuvent ou non être insaturés. Ils résultent de l'oligomérisation (en particulier de la dimérisation) d'acides gras insaturés tels que les acides undécylénique, myristoléique, palmitoléique, oléique, linoléique, linolénique, ricinoléique, eicosénoïque et docosénoïque, que l'on trouve habituellement dans les huiles de pin (Tall oil fatty acids), colza, maïs, tournesol, soja, pépins de raisin, lin, jojoba, ainsi que les acides eicosapentaénoïque et docosahexaénoïque que l'on trouve dans les huiles de poissons.

On peut ainsi utiliser un mélange d'oligomères d'acides gras contenant au moins 30% en poids, voire au moins 50% en poids et de préférence au moins 70% en poids, de dimères d'acides gras en C₁₈ linéaires ou cycliques, éventuellement partiellement ou totalement hydrogénés, ledit mélange contenant un faible pourcentage de monomères d'acides gras (typiquement moins de 5%, de préférence moins de 2% et plus préférentiellement moins de 1% en poids au total) et renfermant de préférence différents isomères d'un même dimère d'acide gras.

On peut citer, comme exemples de mélanges contenant des dimères d'acides gras, ceux commercialisés par la société CRODA sous les dénominations commerciales Pripol® 1006, 1009, 1012, 1013, 1017, 1022, 1025 et 1027, par la société ARIZONA CHEMICALS sous la dénomination commerciale Unidyme® 14, par la société BASF sous la dénomination commerciale Empol® 1008, 1016 ou 1018, ou encore par la société OLEON sous la dénomination commerciale Radiacid® 0980.

On peut également utiliser selon l'invention les diacides et polyacides dérivés de corps gras tels que décrits dans le document FR2962131 A1 et représentés sur les figures (IIa) (IIb) et (IIc) dudit document. Ces diacides sont obtenus par modification d'acides gras ou de mélanges d'acides gras d'origine naturelle (huile de colza, acide oléique par exemple) à l'aide de thiols porteurs d'une fonction acide, par chimie thiol-ène.

On utilise de préférence dans la présente invention un diacide aromatique, tel que l'acide téréphtalique, ou un acide dicarboxylique linéaire et saturé renfermant de 4 à 12 atomes de carbone, tel que l'acide adipique ou l'acide sébacique.

### Diamine

La diamine peut être choisie parmi tout composé linéaire, ramifié ou cyclique, saturé ou insaturé, portant deux fonctions amine primaire. Dans la présente description, le terme « diamine » englobe ainsi notamment les polyamines ne comportant que deux fonctions amines primaires et au moins une autre fonction amine secondaire ou tertiaire.

La diamine peut ainsi être un composé de formule (II) :

H₂N-(CH₂)ₚ-NH₂ (II)

où p est un nombre entier allant de 3 à 20, tel que la cadavérine, la putrescine, l'hexaméthylène diamine ou le 1,12-diaminododécane.

En variante, on peut utiliser des diamines cycloaliphatiques et des diamines à chaîne ramifiée telles que l'isophorone diamine ou la bis(3-méthyl-4-amino cyclochexyl)méthane (BMACM) ou encore les dimères diamines issues d'acides gras végétaux, en particulier d'acides gras en C₁₈, qui peuvent être partiellement ou totalement hydrogénés, tels que ceux mentionnés précédemment. Ces dimères diamines sont notamment disponibles auprès de la société CRODA, par exemple sous la dénomination commerciale Priamine® 1074 ou 1073.

D'autres exemples de diamines sont les diamines linéaires, comportant des hétéroatomes (N) dans leur chaîne, notamment les composés de formule (III) :

H₂N-(CHR₁)ₘ-[NH-(CH₂)ₓ]_{y}-NH-(CHR₂)ₙ-NH₂ (III)

dans laquelle :
R₁ et R₂ désignent indépendamment un atome d'hydrogène ou un groupe alkyle en C₁-C₆ tel qu'un groupe méthyle,
m et n désignent indépendamment un nombre entier allant de 1 à 3,
x désigne un nombre entier allant de 1 à 6,
y désigne un nombre entier allant de 0 à 2.

Dans la formule (III) ci-dessus, au moins l'une, et de préférence toutes, les conditions ci-dessous sont satisfaites :
- R₁ et R₂ désignent un atome d'hydrogène,
- m + n est égal à 2, 3 ou 6, de préférence à 2,
- x désigne un nombre entier allant de 2 à 4,
- y est égal à 0 ou 1, de préférence à 0.

Des exemples préférés de polyamines de formule (III) sont la DETA (diéthylène triamine), la TETA (triéthylène tétramine), la TEPA (tétraéthylène pentamine), la spermine et la dihexylène triamine.

Un autre type encore de diamines, comportant des hétéroatomes (O) dans leur chaîne et utilisables selon l'invention, est constitué des polyétheramines en particulier constituées d'une chaîne polyéther linéaire ou ramifiée, telle qu'une chaîne polypropylène glycol, polyéthylène glycol, ou polytétraméthylène glycol, et leurs copolymères, dont chaque extrémité porte un groupe amine primaire. De tels composés sont notamment disponibles auprès de la société HUNTSMAN sous les dénominations commerciales Jeffamine® Série D, ED ou EDR.

On peut également utiliser selon l'invention des diamines et polyamines à la fois ramifiées et porteuses d'un hétéroatome (S) dans leur chaîne, telles que celles décrites dans le document FR2962131 A1 et représentées sur les figures (IIa) (IIb) et (IIc) dudit document. Ces diamines sont obtenues notamment par modification de triglycérides insaturés d'origine naturelle (huile de colza par exemple) à l'aide de thiols porteurs d'une fonction amine (comme la cystéamine), par chimie thiol-ène.

En variante, les diamines linéaires, les diamines linéaires comportant des hétéroatomes (O, S ou N) dans leur chaîne, les diamines à chaîne ramifiée et les diamines cycloaliphatiques pourront être utilisées en mélanges dans lesquels, de préférence, la fraction molaire de diamines linéaires ne comportant pas d'hétéroatome dans leur chaîne n'excède pas 50% du nombre total de moles de diamines mises en jeu.

Comme dans le cas du diacide, la diamine selon l'invention peut éventuellement être utilisée en mélange avec des mono-amines ou des polyamines, ces mélanges étant choisis de telle sorte que le polycondensat obtenu reste en-deçà du point de gel chimique.

On préfère selon l'invention utiliser des diamines comportant une séquence flexible réputée peu cristallisable. Ces amines peuvent être sélectionnées dans la liste suivante : les polyétheramines comportant une chaine polyéther linéaire ou ramifiée (notamment de structure poly(éthylène glycol), poly(propylène glycol), poly(tétraméthylène glycol) et leurs copolymères), dont chaque extrémité porte un groupe amine primaire, les diamines et polyamines ramifiées dont la chaîne comporte un atome de soufre, et les dimères diamines issues d'acides gras végétaux éventuellement partiellement ou totalement hydrogénés, telles que décrites ci-dessus.

Les dimères diamines issues d'acides gras végétaux éventuellement partiellement ou totalement hydrogénés, sont les diamines préférées.

### Composé modificateur

Dans le procédé selon l'invention, la diamine ou l'acide dicarboxylique réagit avec un composé modificateur portant, d'une part, un groupe associatif comprenant un hétérocycle azoté et, d'autre part, une fonction réactive susceptible de réagir avec la diamine ou le diacide, respectivement.

Ce composé modificateur a comme formule générale : A-L₁-CO-L₂-W où : A est un groupe associatif comprenant un hétérocycle azoté ; L₁ est une liaison chimique ou un bras espaceur constitué d'une chaîne hydrocarbonée saturée ou insaturée, cyclique ou non, éventuellement interrompue par un ou plusieurs atomes d'oxygène et/ou d'azote ; L₂ est une chaîne hydrocarbonée saturée ou insaturée, cyclique ou non, renfermant au moins 4 atomes de carbone, éventuellement interrompue par un ou plusieurs groupes oxo et éventuellement substituée par un ou plusieurs groupes -OH et/ou un ou plusieurs atomes de chlore ; et W est un groupe réactif capable de réagir avec des fonctions amines ou acides de la diamine ou du diacide, respectivement.

Des groupes associatifs particuliers sont les suivants : 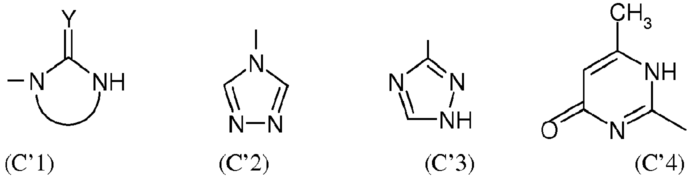 où :
Y est choisi parmi un atome d'oxygène, de soufre ou un groupe NH, et
la liaison représentée par un arc de cercle dans C'1 est choisie parmi -CH₂-CH₂-, -CH=CH- et -NH-CH₂-.

Le groupe associatif présent dans le composé modificateur utilisé dans l'invention est choisi parmi les groupes imidazolidonyle, triazolyle, triazinyle, bis-uréyle et uréido-pyrimidyle. Un groupe associatif préféré pour une utilisation dans la présente invention est le groupe imidazolidonyle.

Dans une forme d'exécution préférée de l'invention, le composé modificateur réagit avec les fonctions amines de la diamine. Dans ce cas, le rapport molaire des fonctions acides du diacide aux fonctions réactives du composé modificateur est compris entre 1 et 8, par exemple entre 1 et 3, étant entendu que le nombre de moles de fonctions amines de la diamine est égal à la somme du nombre de moles des fonctions acides et réactives précitées.

Des exemples de tels composés modificateurs sont ceux de formule A-L₁-CO-L₂-W₁
où :
A, L₁ et L₂ ont les significations indiquées précédemment, et
W₁ est une fonction acide carboxylique, ester, anhydride ou chlorure d'acyle.

Selon une forme d'exécution préférée de l'invention, le composé modificateur répond à la formule (IV) : 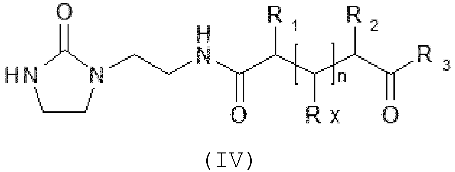 dans laquelle : R₁, R₂ et chacun des groupes Rx désignent indépendamment un atome d'hydrogène, un groupe -OH ou un groupe -CH₃, de préférence un atome d'hydrogène ; n va de 2 à 12 et est de préférence égal à 2, 4 ou 6, plus préférentiellement à 2 ; et R₃ désigne un groupe -OH, un groupe -OCH₃ ou un atome de chlore, de préférence un groupe -OCH₃.

Plus préférentiellement encore, le composé modificateur répond à la formule suivante (V) : 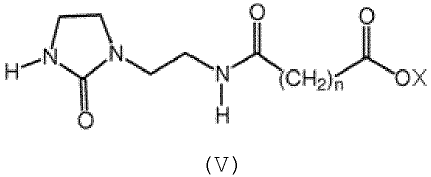 où n est compris entre 4 et 14 et X = H ou CH₃.

Les composés modificateurs précités peuvent être obtenus en faisant réagir de l'UDETA (ou 2-aminoéthyl)imidazolidin-2-one) sur :
- un anhydride alicyclique, tel que l'anhydride glutarique, succinique, maléique, citraconique, diglycolique ou itaconique, ou
- un anhydride aromatique, tel que les anhydrides 1,8-naphthalique, phtalique et isatoïque, ou
- un acide dicarboxylique, tel que l'acide adipique, ou
- un diester d'acide carboxylique, tel que l'adipate de diméthyle ou le téréphtalate de diméthyle, ou
- un chlorure d'acide dicarboxylique.

L'UDETA peut elle-même être préparée par réaction de l'urée avec la diéthylène triamine (DETA). D'autres composés modificateurs similaires peuvent être obtenus en remplaçant l'UDETA par l'UTETA ou l'UTEPA, qui peuvent respectivement être préparées en faisant réagir de l'urée sur la triéthylène tétramine (TETA) et la tétraéthylène pentamine (TEPA).

D'autres composés modificateurs répondent à la formule (VI) : 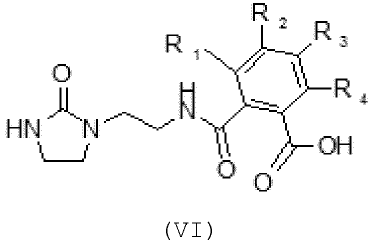 dans laquelle : R₁, R₂, R₃ et R₄ désignent indépendamment un atome d'hydrogène, un groupe hydroxy, un groupe amino, un groupe nitro ou un groupe alkyle, ainsi que leurs isomères de position et leurs esters, chlorures d'acyle et anhydrides.

Comme indiqué précédemment, le composé modificateur peut en variante réagir avec les fonctions acides de l'acide dicarboxylique utilisé dans le procédé selon l'invention. Dans ce cas, le rapport molaire des fonctions amines de la diamine aux fonctions réactives du composé modificateur est compris entre 1 et 8, étant entendu que le nombre de moles de fonctions acides du diacide est égal à la somme du nombre de moles des fonctions amines et réactives précitées.

Dans ce mode de réalisation, le composé modificateur a généralement pour formule : A-L₁-CO-L₂-W₂
où :
A, L₁ et L₂ ont les significations indiquées précédemment, et
W₂ est un groupe OH ou NH₂.

Un exemple d'un tel composé modificateur répond à la formule (VII) : 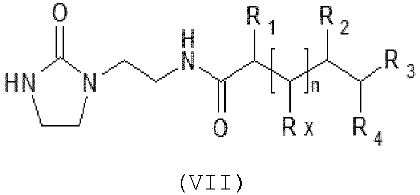 dans laquelle : R₁, R₂, Rx et n ont les significations données précédemment, R₃ désigne un groupe OH ou NH₂ et R₄ désigne un atome d'hydrogène ou un groupement alkyle.

Un autre composé modificateur utilisable dans ce mode de réalisation est le dérivé d'uréido-pyrimidyle (UPy) suivant : 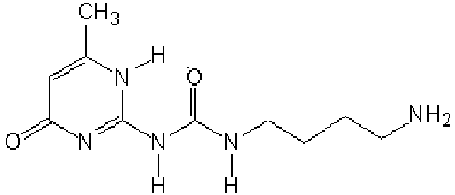 dont la synthèse est décrite dans l'exemple 1 du document FR 2 954 941.

Comme indiqué précédemment, dans les deux formes d'exécution de l'invention mentionnées ci-dessus, c'est à dire ***(i)*** lorsque le composé modificateur réagit avec les fonctions amines de la diamine et ***(ii)*** lorsque le composé modificateur réagit avec les fonctions acides du diacide, le diacide peut être remplacé par un mélange de monoacide B1, diacide B2, triacide B3 etc..., et/ou la diamine peut être remplacée par un mélange de monoamine A1, diamine A2, triamine A3.... Dans ces cas, les quantités de polyacides et polyamines de fonctionnalité supérieure ou égale à 3 doivent être limitées de façon à ne pas atteindre le point de gel chimique.

Pour cela, les nombres de moles de chaque composant des mélanges sont choisis de façon à ce que le produit P suivant reste strictement inférieur à 1 : 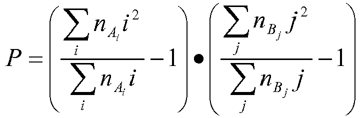 où :
*n_{A1}* désigne, dans le cas ***(i)*** le nombre de moles de monoamine et dans le cas ***(ii)*** la somme des nombres de moles de monoamine et de composé modificateur,
*n_{B1}* désigne, dans le cas ***(i)*** la somme des nombres de moles de monoacide et de composé modificateur et dans le cas ***(ii)*** le nombre de moles de monoacide,
*n_{A2}* désigne le nombre de moles de diamine,
*n_{B2}* désigne le nombre de moles de diacide,
*n_{A3}* désigne le nombre de moles de triamine,
*n_{B3}* désigne le nombre de moles de triacide,
*n_{Ai}* (i>3) désigne le nombre de moles de polyamine comportant i fonctions amine,
*n_{Bj}* (j>3) désigne le nombre de moles de polyacide comportant j fonctions acides.

### Synthèse du matériau supramoléculaire

Dans le procédé selon l'invention, on utilise un composé modificateur qui a été préalablement synthétisé, isolé et éventuellement purifié de façon à présenter une pureté d'au moins 90%. La synthèse de ce composé peut éventuellement constituer une étape préliminaire du procédé selon l'invention. En revanche, il est exclu que ce composé soit synthétisé *in situ*, pour éviter la formation de produits indésirables tels que celui illustré ci-dessous, obtenu à partir d'UDETA et d'un diacide carboxylique linéaire :

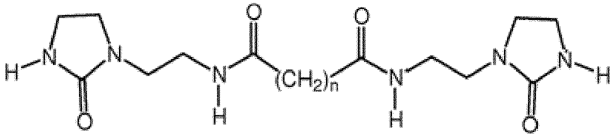

L'acide dicarboxylique, la diamine et le composé modificateur sont introduits, dans les rapports molaires indiqués précédemment, soit simultanément, soit successivement dans un ordre quelconque, dans un réacteur. La réaction de polycondensation est effectuée à une température de 50 à 200°C, par exemple de 160 à 200°C, pendant une durée allant de 1 à 24 heures, notamment de 4 à 6h. La réaction est habituellement effectuée sous agitation, par exemple à une vitesse de 200 à 350 tours/min. Elle est de préférence conduite en l'absence de solvant et avantageusement sous un flux de gaz inerte, tel que de l'azote, permettant d'évacuer l'eau et le méthanol produits lors de la réaction et de déplacer ainsi celle-ci vers la formation des oligo-amides recherchés.

L'évolution de la réaction peut être suivie par spectroscopie infrarouge. La disparition des bandes caractéristiques du diacide ou de la diamine au profit de l'amide permet de déterminer le moment où la réaction est achevée.

Cette réaction produit des oligo-amides de longueurs de chaîne variables, fonctionnalisés à chacune de leurs extrémités par des motifs associatifs et donc susceptibles de s'associer les uns aux autres par des liaisons hydrogène réversibles en fonction de la température. Il a été observé que la longueur des chaînes obtenues, ainsi que leur fraction cristallisable, étaient directement liées aux rapports stoechiométriques précités. Ces derniers sont donc ajustés afin de permettre l'obtention d'un matériau ayant la cristallinité requise pour lui conférer de bonnes propriétés mécaniques et une masse moléculaire suffisamment faible pour présenter une faible viscosité à l'état fondu.

Le polycondensat obtenu à l'issue de ce procédé est semi-cristallin, caractérisé par exemple par une enthalpie de fusion supérieure à 10 J/g, et il a généralement une température de fusion (T_{f}) comprise entre 120 et 260°C, de préférence entre 130 et 180°C, et plus préférentiellement entre 140 et 170°C, et une température de transition vitreuse (T_{g}) généralement comprise entre -25°C et 100°C et de préférence entre - 25°C et 10°C.

Sa masse moléculaire moyenne en nombre, telle que mesurée par GPC, est généralement inférieure à 4.500 g/mol, par exemple comprise entre 1.000 et 3.000 g/mol, mais elle peut en variante aller jusqu'à 10.000 g/mol.

Ce matériau présente une faible viscosité à l'état fondu, généralement inférieure à 10 Pa.s, de préférence inférieure à 1 Pa.s et typiquement comprise entre 0,1 et 0,5 Pa.s, à 30°C au-dessus de son point de fusion, et de bonnes propriétés mécaniques à l'ambiante et dans une large gamme de température au-dessus de l'ambiante, qui se traduisent par une contrainte à la rupture supérieure à 1 MPa, notamment supérieure à 3 MPa, de préférence supérieure à 4 MPa, voire supérieure à 10 MPa, et éventuellement un comportement ductile en traction.

Les propriétés précitées sont mesurées suivant les techniques données dans la partie Exemples de cette description. Le matériau obtenu suivant le procédé selon l'invention présente au moins l'une, et de préférence toutes, ces propriétés.

Le matériau obtenu suivant le procédé selon l'invention peut notamment être utilisé pour fabriquer des joints d'étanchéité, des isolants thermiques ou acoustiques, des semelles de chaussures, des emballages, des revêtements (peintures, films, produits cosmétiques), des systèmes de piégeage et relarguage d'actifs, des tubes à vide, et d'une manière générale des pièces devant présenter de bonnes résistance à la déchirure et/ou à la fatigue, des additifs rhéologiques, des additifs pour le bitume ou des additifs pour colles thermofusibles, ou encore des matrices de composites.

L'invention sera mieux comprise à la lumière des exemples suivants, donnés à des fins d'illustration seulement et qui n'ont pas pour but de restreindre la portée de l'invention, définie par les revendications annexées.

### EXEMPLES

### Méthodes de mesure

1. Analyse thermique : les échantillons ont été caractérisés par analyse calorimétrique différentielle à balayage (DSC). Le protocole suivant a été appliqué : premier chauffage à 10°C/min de -100°C à 250°C, isotherme de 5 min à 250°C, refroidissement à -10°C/min jusqu'à -100°C, isotherme à -100°C pendant 5 min puis second chauffage jusqu'à 250°C à 10°C/min. Les enthalpies de fusion ont été mesurées à l'aide du logiciel TA Universal Analysis en mode « Sigmoidal tangent ». Les températures de fusion ont été déterminées au sommet du pic et les températures de transition vitreuse au point d'inflexion.
2. Rhéologie : la viscosité des échantillons a été mesurée avec un taux de cisaillement de 100s⁻¹, à l'aide d'un rhéomètre équipé d'une géométrie cône-plan de diamètre 50 mm. L'échantillon était placé sous la géométrie préchauffée à 180°C.
3. Propriétés mécaniques : les propriétés en traction uniaxiale ont été étudiées sur des échantillons en forme d'haltères de 12 mm x 2 mm x 1,2 mm, à l'aide d'une machine de traction INSTRON 5564. Les essais ont été effectués à température ambiante, avec une vitesse de 2 mm/min. Trois tests indépendants ont été réalisés pour chaque matériau et la moyenne des résultats de ces trois tests a ensuite été calculée.

### Exemple 1 : Préparation de composés modificateurs selon l'invention

Cet exemple illustre la synthèse de différents composés modificateurs utilisés selon l'invention.

### Exemple 1A : ouverture d'anhydride

Le schéma réactionnel était le suivant :

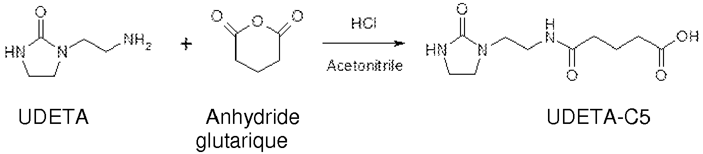

Dans un premier temps, l'anhydride glutarique (7,95 g soit 0,07 mol) a été introduit dans une ampoule à addition. L'acétonitrile (15ml) a été ajouté à l'anhydride et solubilisé à 60°C sous agitation (la solubilisation n'était pas totale). Une solution d'UDETA (10 g soit 0,077 mol) dans l'acétonitrile (15 ml) a été préparée et introduite dans un ballon bicol muni d'un barreau aimanté et surmonté de l'ampoule à addition. Le ballon a été introduit dans un bain d'eau à température ambiante, quelques gouttes d'acide chlorhydrique à 36-38% (soit 0,001 mol) ont ensuite été ajoutées et la solution d'anhydride a été introduite goutte à goutte sur une période de 30 minutes. Le mélange réactionnel a été maintenu sous agitation durant 20h à température ambiante, puis à 40°C durant 4 heures. Le produit, qui précipitait au fur et à mesure de sa formation sous forme de poudre blanche, a été récupéré par filtration (pompe à vide), lavé 2 fois à l'acétonitrile et séché sous cloche à température ambiante. Le produit obtenu était une poudre blanche caractérisée chimiquement : la pureté du produit final a pu être aisément déterminée par RMN du proton.

Ce procédé peut être appliqué à la synthèse de composés modificateurs similaires, ayant des tailles de chaînes différentes.

### Exemple 1B : condensation d'UDETA sur un diester

Le schéma réactionnel était le suivant :

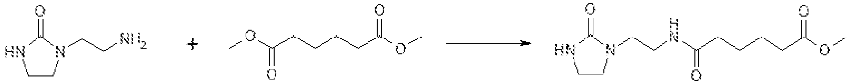

Dans un ballon bicol muni d'un barreau aimanté ont été introduits l'UDETA (30 g soit 0,232 mol) et l'adipate de diméthyle (364,13 g soit 2,090 mol) en large excès (9 equivalents). Le mélange de départ transparent a été mis sous agitation et un flux d'azote a été mis en place afin d'évacuer le méthanol qui se formait dans le milieu réactionnel. Le ballon a été placé dans un bain d'huile silicone chauffé à 140°C pendant une période de 6 heures. En fin de réaction, l'excès de diester a été évacué par distillation à 160°C sous vide statique au départ, à 180°C sous vide statique lorsque la distillation ralentissait, puis sous vide dynamique à 160°C pour récupérer la plus grande quantité possible de diester. Enfin, le produit formé a été lavé cinq fois avec du pentane et séché sous cloche à température ambiante durant 24 heures. Le produit final (UDETA-C6) a pu être caractérisé par RMN et présentait une bonne pureté.

Un procédé similaire peut être mis en oeuvre en substituant un acide dicarboxylique ou un chlorure d'acide dicarboxylique au diester utilisé ci-dessus.

### Exemple 1C : ouverture de lactone

Le schéma réactionnel était le suivant :

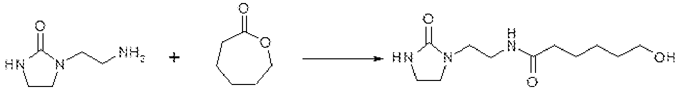

Dans un premier temps, l'UDETA (20 g soit 0,155 mol) a été solubilisée dans 30 ml d'acétonitrile dans un ballon bicol muni d'un barreau aimanté à température ambiante. Le ballon était surmonté d'une ampoule à addition dans laquelle a été introduite une solution de 18,3 ml (0,17 mol) de caprolactone dans 15 ml d'acétonitrile. Cette solution a été ajoutée goutte à goutte dans le mélange réactionnel sur une période de 30 minutes à température ambiante. Le mélange a ensuite été laissé sous agitation durant 12 heures, puis 4 heures supplémentaires à 40°C. La solution a été concentrée à l'évaporateur rotatif et mise au congélateur durant 12 heures afin de cristalliser le produit de réaction qui a ensuite été récupéré par filtration, lavé à l'acétonitrile et séché sous cloche durant 6 heures. Le produit de réaction a pu être facilement analysé par RMN du proton pour en déterminer la pureté.

Une grande variété de lactones peuvent être ouvertes de la même façon et donner lieu à des chaînes carbonées fonctionnalisées.

### Exemple 2 : Préparation d'un oligo-amide supramoléculaire à base de dimère d'acide

On a condensé un diacide gras insaturé (Pripol® 1009), une diamine comportant un cycle et une insaturation (Priamine® 1074) et un composé modificateur obtenu tel que décrit à l'Exemple 1B (UDETA-C6), pour obtenir les composés de formule suivante : 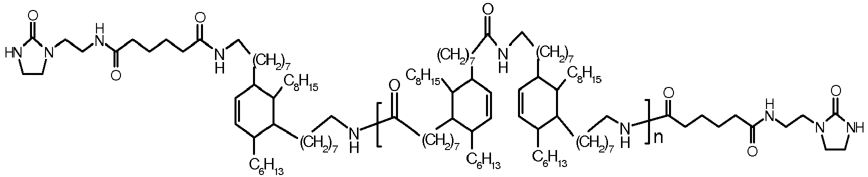 où n variait de 0 à 3.

La réaction a été effectuée dans un réacteur double paroi de diamètre 60 mm et de volume nominal de 1L, régulé à l'aide d'un bain thermostaté à circulation d'huile siliconée dont les tuyaux étaient renforcés par une gaine métallique. Ce réacteur était surmonté d'un agitateur mécanique et était pourvu d'une vanne de fond, d'une entrée de gaz et d'un bulleur. La réaction était suivie par infrarouge et les produits de réaction ont été analysés par RMN et GPC.

Le produit correspondant à n = 1 a ainsi été synthétisé de la manière suivante.

52,5 g (0,18 mol) de Pripol® 1009 ont été pesés et introduits dans le réacteur. On a ensuite introduit dans le réacteur 101,01 g (0,37 mol) de Priamine® 1074 puis 50 g (soit 0,18 mol) d'UDETA-C6. Le réacteur a alors été fermé et chauffé à 180°C sous une agitation de 280 tours/minute. Un flux d'azote de 300 ml/minute a été mis en place à l'aide d'un tuyau pouvant résister à de hautes températures. Ce tuyau a été introduit au plus près du mélange sous agitation. Une sortie de gaz reliée à un bulleur permettait de vérifier l'étanchéité du réacteur et de retenir le méthanol formé au cours de la réaction. Un suivi par infrarouge (IR) a été mis en place pour déterminer le moment où le chauffage devait être arrêté. L'analyse en spectroscopie IR confirmait la disparition de la bande v (C=O) de la fonction carboxylate vers 1394 cm⁻¹ et l'apparition de la bande v(C=O) de l'amide vers 1650 cm⁻¹. La réaction était complète au bout de 6 heures et l'oligomère obtenu a été récupéré au niveau de la vanne de fond dans un bécher en téflon. Le produit obtenu cristallisait assez rapidement dans le bécher et n'y adhérait pas. Il a ainsi pu être facilement être récupéré et analysé.

Tous les échantillons de cet exemple ont été obtenus de la même manière. Les produits différaient par leurs tailles de chaînes, qui dépendait elle-même de la valeur de n, c'est-à-dire du rapport molaire des fonctions acides du diacide aux fonctions réactives du composé modificateur. Deux exemples comparatifs ont en outre été réalisés, en utilisant des composés modificateurs qui n'entrent pas dans le champ de la présente invention. Enfin, un exemple supplémentaire a été réalisé en substituant le Pripol® 1017 au Pripol® 1009. Les différents essais réalisés sont rassemblés dans le tableau ci-dessous.

| **n=0** | M (g/mol) | n (mol) | m (g) | Mn (g/mol) |
|---|---|---|---|---|
| Pripol 1009 | 285 | 0 | 0.0 | 1026 |
| Priamine | 274 | 0.184325 | 50.5 | |
| UDETA-C6 | 271.26 | 0.184325 | 50 | |

| **n=1** | M (g/mol) | n (mol) | m (g) | Mn (g/mol) |
|---|---|---|---|---|
| Pripol 1009 | 285 | 0.184325 | 52.5 | |
| Priamine | 274 | 0.36865 | 101.0 | 2108 |
| UDETA-C6 | 271.26 | 0.184325 | 50 | |

| **n=2** | M (g/mol) | n (mol) | m (g) | Mn (g/mol) |
|---|---|---|---|---|
| Pripol 1009 | 285 | 0.03985 965 | 11.4 | |
| Priamine | 274 | 0.05978 947 | 16.4 | 3190 |
| UDETA-C6 | 271.26 | 0.01992 982 | 5.40616 421 | |

| **n=3** | M (g/mol) | n (mol) | m (g) | Mn (g/mol) |
|---|---|---|---|---|
| Pripol 1009 | 285 | 0.04568 421 | 13.0 | |
| Priamine | 274 | 0.06091 228 | 16.7 | 4272 |
| UDETA-C6 | 276.26 | 0.01522 807 | 4.2 | |

| **Contre exemple 2-1** | M (g/mol) | n (mol) | m (g) | Mn (g/mol) |
|---|---|---|---|---|
| Pripol 1009 | 285 | 0.008394 16 | 2.41 | |
| Priamine | 274 | 0.016788 32 | 4.60 | 1911 |
| Mono-lauroyl adipamide* | 327.51 | 0.008394 16 | 2.75 | |
| *produit de la réaction de la dodécylamine avec l'adipate de diméthyle | | | | |

| **Contre exemple 2-2** | M (g/mol) | n (mol) | m (g) | Mn (g/mol) |
|---|---|---|---|---|
| Pripol 1009 | 285 | 0.00930 657 | 2.46 | |
| Priamine | 274 | 0.01861 314 | 5.10 | 1656 |
| Acide laurique | 200.32 | 0.00930 657 | 1.86 | |

| | M (g/mol) | n (mol) | m (g) | Mn (g/mol) |
|---|---|---|---|---|
| Pripol 1017 | 288,66 | 0,02764498 | 7,98 | 2080 |
| Priamine | 274 | 0,20189781 | 55,32 | |
| UDETA-C6 | 271,26 | 0,02764875 | 7,50 | |

### Exemple 3 : Préparation d'un oligo-amide supramoléculaire à base d'acide adipique

Une seconde série d'échantillons a été préparée de manière similaire à l'Exemple 2, en remplaçant le Pripol® 1009 par l'acide adipique, pour obtenir des matériaux répondant à la formule suivante : 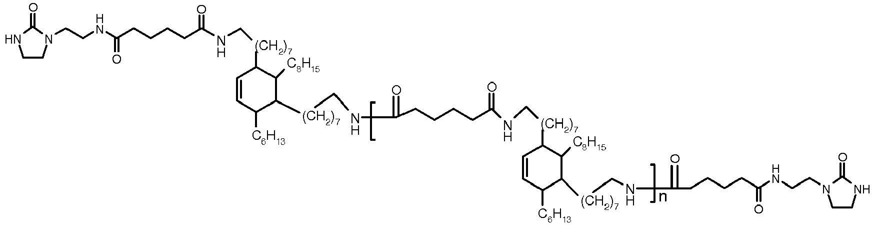 où n variait de 0 à 3.

Comme dans l'Exemple 2, un échantillon comparatif a également été préparé en remplaçant l'UDETA-C6 par un composé modificateur n'entrant pas dans le champ de l'invention. Les conditions de préparation des échantillons de cette série sont résumées dans le tableau ci-dessous :

| **n=0** | M (g/mol) | n (mol) | m (g) | Mn (g/mol) |
|---|---|---|---|---|
| Acide adipique | 72.06 | 0 | 0.00 | |
| Priamine | 274 | 0.184325 | 50.50 | 1026 |
| UDETA-C6 | 271.26 | 0.184325 | 50.00 | |

| **n=0,33** | M (g/mol) | n (mol) | m (g) | Mn (g/mol) |
|---|---|---|---|---|
| Acide adipique | 72.06 | 0.04204 | 3.03 | |
| Priamine | 274 | 0.16819 | 46.11 | 1387 |
| UDETA-C6 | 271.26 | | | |

| **n=1** | M (g/mol) | n (mol) | m (g) | Mn (g/mol) |
|---|---|---|---|---|
| Acide adipique | 72.06 | 0.042394 75 | 3.09 | |
| Priamine | 274 | 0.084789 5 | 23.27 | 1062 |
| UDETA-C6 | 271.26 | 0.042394 75 | 11.50 | |

| **n=2** | M (g/mol) | n (mol) | m (g) | Mn (g/mol) |
|---|---|---|---|---|
| Acide adipique | 72.06 | 0.03880 779 | 2.80 | 1372 |
| Priamine | 274 | 0.05821 168 | 15.95 | |
| UDETA-C6 | 271.26 | 0.01940 389 | 5.26 | |

| **n=3** | M (g/mol) | n (mol) | m (g) | Mn (g/mol) |
|---|---|---|---|---|
| Acide adipique | 72.06 | 0.04125 | 3.00 | |
| Priamine | 274 | 0.055 | 15.07 | 1693 |
| UDETA-C6 | 276.26 | 0.01375 | 3.80 | |
| | | | | |

| **Contre exemple 3-1** | M (g/mol) | n (mol) | m (g) | Mn (g/mol) |
|---|---|---|---|---|
| Acide adipique | 72.06 | 0.03880 779 | 2.82 | 1201 |
| Priamine | 274 | 0.05821 168 | 15.95 | |
| Mono-lauroyl adipamide* | 185.35 | 0.01940 389 | 3.60 | |

| | | | | |
|---|---|---|---|---|
| *produit de la réaction de la dodécylamine avec l'adipate de diméthyle | | | | |

### Exemple 4 : Evaluation des propriétés des matériaux supramoléculaires

Les matériaux préparés aux Exemples 2 et 3 ont été soumis aux différents tests présentés plus haut, en vue d'évaluer leurs propriétés physico-chimiques et mécaniques. Les matériaux synthétisés selon l'exemple 3 ont été recuits pendant 1h à 100°C avant mesure de leur enthalpie de fusion.

Les résultats de ces essais sont rassemblés dans le tableau ci-dessous :

| Ex. 2 | n | Mn théo (g/mol) | Tg (°C) | Tf (°C) | η (180°C, 100 s⁻¹) | Contrainte rupt. (MPa) | Allong. Rupt. (%) | ΔH fus. (J/g) |
|---|---|---|---|---|---|---|---|---|
| | 0 | 1026 | 1.3 | 164 | 0.08 | / | / | 41 |
| | 0.33 | 1387 | 3.2 | 163 | 0.12 | / | / | 26 |
| | 1 | 2108 | -9.3 | 160 | 0.16 | 4.6 | 30 | 25 |
| | 2 | 3190 | -16.6 | 155.6 | 0.25 | 1.8 | 36 | 15 |
| | 3 | 4272 | -18 | 153.2 | 0.39 | 1.3 | 31 | 6.2 |
| Contre -ex. 2-1 | 1 | 3303 | -24.7 | 107 | 0.08 | 2.3 | 15 | 6.2 |
| Contre -ex. 2-2 | 1 | 3048 | / | 21 | 0.08 | Liquide visqueux | / | / |

| Ex. 3 | n | Mn théo (g/mol) | Tg (°C) | Tf (°C) | η (180°C, 100 s⁻¹) | Contrainte rupt. (MPa) | Elong. Rupt. (%) | ΔH fus. (J/g) |
|---|---|---|---|---|---|---|---|---|
| | 0 | 1026 | 3.3 | 166 | 0.08 | / | / | 41 |
| | 1 | 1683 | 6.1 | 155.8 | | 7 | 7.7 | 27.08 |
| | 2 | 2339 | 9 | 156 | 0.21 | 7.6 | 24.7 | 21 |
| | 3 | 3005 | -4.6 | 149 | | 12.2 | 28 | 12.37 |
| Contre -ex. 3-1 | 1 | 1403 | -26.2 | 111.9 | 0.19 | 3.8 | 8 | 22 |

Il ressort de ces essais que le remplacement du composé modificateur selon l'invention par un autre composé ne comportant pas d'hétérocycle azoté résulte en un matériau ayant un point de fusion inférieur à 120°C, qui est insuffisant pour de nombreuses applications, voire en un matériau qui n'est pas solide mais se présente sous forme d'un liquide visqueux à température ambiante. En outre, les matériaux préparés en l'absence de diacide (n=0) ou en présence d'une quantité insuffisante de diacide (n = 0,33) étaient trop fragiles pour être démoulés et n'ont donc pas pu être testés.

En outre, il a été observé que les propriétés ci-dessus des matériaux selon l'invention étaient similaires à celles obtenues en utilisant un polyamide-6 typique, de poids moléculaire égal à 18.000 g/mol. Toutefois, leur viscosité à l'état fondu est plus de 100 fois inférieure à celle de ce type de polymères, ce qui facilite grandement leur mise en oeuvre.

### Exemple 5 (comparatif) : Matériaux obtenus en remplaçant le composé modificateur par l'UDETA

On a préparé un oligo-amide comparatif, en utilisant UDETA au lieu du groupe modificateur UDETA-C6 selon l'invention. Ce matériau a été préparé suivant un procédé similaire à celui indiqué à l'Exemple 2, à partir de 50 g de Pripol® 1009 (0.175 mol), 24 g de Priamine® 1074 (0.088 mol) et 11.32 g d'UDETA (0.088 mol), excepté que la Priamine® et l'UDETA ont été introduits dans le réacteur chauffé à 180°C avant d'ajouter le Pripol® 1009.

Le produit obtenu se présentait, à l'ambiante, sous la forme d'un liquide très visqueux ne présentant aucune propriété mécanique significative. Sa viscosité était inférieure à 0.3 Pa.s à 180°C.

## Revendications

1. Procédé de préparation d'un matériau comprenant des oligo-amides X.Y linéaires ou ramifiés, terminés à plus de 90% en nombre de leurs extrémités par un même groupe -M-L₂-CO-L₁-A dans lequel M est choisi parmi les groupes CO, NH et O, ledit procédé comprenant une étape de polycondensation :
(a) d'au moins un acide dicarboxylique,
(b) d'au moins une diamine, et
(c) d'au moins un composé modificateur de formule A-L₁-COL₂-W, préalablement synthétisé et isolé, où : A est un groupe associatif comprenant un hétérocycle azoté ; L₁ est une liaison chimique ou un bras espaceur constitué d'une chaîne hydrocarbonée saturée ou insaturée, cyclique ou non, éventuellement interrompue par un ou plusieurs atomes d'oxygène et/ou d'azote ; L₂ est une chaîne hydrocarbonée saturée ou insaturée, non cyclique, renfermant au moins 4 atomes de carbone, éventuellement interrompue par un ou plusieurs groupes oxo et éventuellement substituée par un ou plusieurs groupes -OH et/ou un ou plusieurs atomes de chlore ; W est un groupe réactif capable de réagir :
- soit avec les fonctions amines de la diamine, le rapport molaire des fonctions acides du diacide aux fonctions réactives du composé modificateur allant de 1 à 8, étant entendu que le nombre de moles de fonctions amines de la diamine est égal à la somme du nombre de moles des fonctions acides et réactives précitées,
- soit avec les fonctions acides du diacide, le rapport molaire des fonctions amines de la diamine aux fonctions réactives du composé modificateur allant de 1 à 8, étant entendu que le nombre de moles de fonctions acides du diacide est égal à la somme du nombre de moles des fonctions amines et réactives précitées.

2. Procédé selon la revendication 1, **caractérisé en ce que** l'acide dicarboxylique est un diacide aromatique, ou un acide dicarboxylique linéaire et saturé, renfermant de 4 à 22 atomes de carbone.

3. Procédé selon la revendication 2 **caractérisé en ce que** l'acide dicarboxylique est l'acide téréphtalique.

4. Procédé selon la revendication 2 **caractérisé en ce que** l'acide dicarboxylique est l'acide adipique.

5. Procédé selon l'une quelconque des revendications précédentes, **caractérisé en ce que** la diamine est choisie parmi les polyétheramines comportant une chaine polyéther linéaire ou ramifiée dont chaque extrémité porte un groupe amine primaire, les diamines et polyamines ramifiées dont la chaîne comporte un atome de soufre, et les dimères diamines issues d'acides gras végétaux éventuellement partiellement ou totalement hydrogénés.

6. Procédé selon la revendication 5 **caractérisé en ce que** la diamine est choisie parmi les dimères diamines issues d'acides gras végétaux éventuellement partiellement ou totalement hydrogénés.

7. Procédé selon l'une quelconque des revendications précédentes **caractérisé en ce que** le groupe associatif A est choisi parmi les groupes imidazolidonyle, triazolyle, triazinyle, bis-uréyle et uréido-pyrimidyle, de préférence imidazolidonyle.

8. Procédé selon l'une quelconque des revendications précédentes, **caractérisé en ce que** le composé modificateur répond à la formule (IV) : 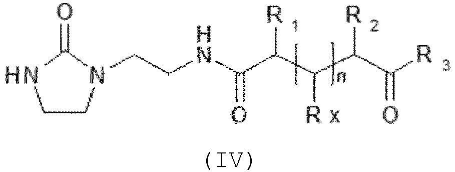 dans laquelle : R₁, R₂ et chacun des groupes Rx désignent indépendamment un atome d'hydrogène, un groupe -OH ou un groupe -CH₃ ; n va de 2 à 12 ; et R₃ désigne un groupe -OH, un groupe -OCH₃ ou un atome de chlore.

9. Procédé selon la revendication 8, **caractérisé en ce que** le composé modificateur répond à la formule suivante (V) : 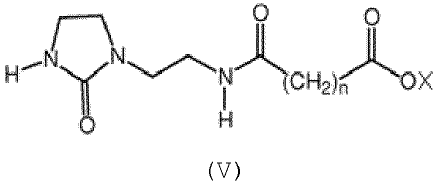 où n est compris entre 4 et 14 et X=H ou CH₃.

## Patentansprüche

1. Verfahren zur Herstellung eines Materials, das lineare oder verzweigte Oligoamide X.Y umfasst, die zahlenmäßig an mehr als 90% ihrer Enden durch die gleiche Gruppe -M-L₂-CO-L₁-A terminiert sind, wobei M aus CO-, NH- und O-Gruppen ausgewählt ist, wobei das Verfahren einen Schritt der Polykondensation
(a) mindestens einer Dicarbonsäure,
(b) mindestens eines Diamins und
(c) mindestens einer vorher synthetisierten und isolierten Modifikatorverbindung der Formel A-L₁-CO-L₂-W, wobei A für eine assoziative Gruppe steht, die einen stickstoffhaltigen Heterocyclus umfasst; L₁ für eine chemische Bindung oder einen Spacer-Arm, der aus einer cyclischen oder nichtcyclischen, gesättigten oder ungesättigten Kohlenwasserstoffkette, die gegebenenfalls durch ein oder mehrere Sauerstoff- und/oder Stickstoffatome unterbrochen ist, besteht, steht; L₂ für eine nichtcyclische gesättigte oder ungesättigte Kohlenwasserstoffkette mit mindestens 4 Kohlenstoffatomen, die gegebenenfalls durch eine oder mehrere Oxogruppen unterbrochen und gegebenenfalls durch eine oder mehrere -OH-Gruppen und/oder ein oder mehrere Chloratome substituiert ist, steht und W für eine reaktive Gruppe steht, die:
- entweder mit den Aminfunktionen des Diamins reagieren kann, wobei das Molverhältnis von Säurefunktionen der Disäure zu reaktiven Funktionen der Modifikatorverbindung im Bereich von 1 bis 8 liegt, wobei es sich versteht, dass die Zahl der Mole von Aminfunktionen des Diamins gleich der Summe der Zahl der Mole der obigen Säurefunktionen und reaktiven Funktionen ist,
- oder mit den Säurefunktionen der Disäure reagieren kann, wobei das Molverhältnis von Aminfunktionen des Diamins zu reaktiven Funktionen der Modifikatorverbindung im Bereich von 1 bis 8 liegt, wobei es sich versteht, dass die Zahl der Mole von Säurefunktionen der Disäure gleich der Summe der Zahl der Mole der obigen Aminfunktionen und reaktiven Funktionen ist,
umfasst.

2. Verfahren nach Anspruch 1, **dadurch gekennzeichnet, dass** es sich bei der Dicarbonsäure um eine aromatische Disäure oder eine lineare und gesättigte Dicarbonsäure mit 4 bis 22 Kohlenstoffatomen handelt.

3. Verfahren nach Anspruch 2, **dadurch gekennzeichnet, dass** es sich bei der Dicarbonsäure um Terephthalsäure handelt.

4. Verfahren nach Anspruch 2, **dadurch gekennzeichnet, dass** es sich bei der Dicarbonsäure um Adipinsäure handelt.

5. Verfahren nach einem der vorhergehenden Ansprüche, **dadurch gekennzeichnet, dass** das Diamin aus Polyetheraminen mit einer linearen oder verzweigten Polyetherkette, wobei jedes Ende davon eine primäre Aminogruppe trägt, verzweigten Diaminen und Polyaminen, wobei jedes Kettenende davon ein Schwefelatom umfasst, und Dimerdiaminen, die sich von gegebenenfalls teilweise oder vollständig hydrierten pflanzlichen Fettsäuren ableiten, ausgewählt wird.

6. Verfahren nach Anspruch 5, **dadurch gekennzeichnet, dass** das Diamin aus Dimerdiaminen, die sich von gegebenenfalls teilweise oder vollständig hydrierten pflanzlichen Fettsäuren ableiten, ausgewählt wird.

7. Verfahren nach einem der vorhergehenden Ansprüche, **dadurch gekennzeichnet, dass** die assoziative Gruppe A aus Imidazolidonyl-, Triazolyl-, Triazinyl-, Bisureyl- und Ureidopyrimidylgruppen, vorzugsweise Imidazolidonyl, ausgewählt wird.

8. Verfahren nach einem der vorhergehenden Ansprüche, **dadurch gekennzeichnet, dass** die Modifikatorverbindung der Formel (IV) entspricht: 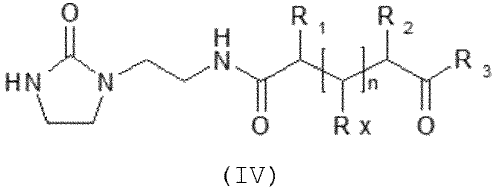 in der: R₁ und R₂ und jede der Gruppen Rx unabhängig für ein Wasserstoffatom, eine -OH-Gruppe oder eine -CH₃-Gruppe stehen; n im Bereich von 2 bis 12 liegt und R₃ für eine -OH-Gruppe, eine -OCH₃-Gruppe oder ein Chloratom steht.

9. Verfahren nach Anspruch 8, **dadurch gekennzeichnet, dass** die Modifikatorverbindung der folgenden Formel (V) entspricht: 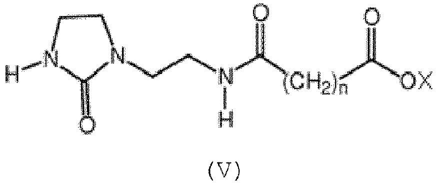 wobei n zwischen 4 und 14 liegt und X = H oder CH₃.

## Claims

1. Process for preparing a material comprising linear or branched oligoamides X.Y, more than 90% by number of the ends of which end with one and the same group - M-L₂-CO-L₁-A in which M is chosen from CO, NH and O groups, said process comprising a step of polycondensation:
(a) of at least one dicarboxylic acid,
(b) of at least one diamine, and
(c) of at least one modifier compound of formula A-L₁-CO-L₂-W, synthesized and isolated beforehand, wherein: A is an associative group comprising a nitrogenous heterocycle; L₁ is a chemical bond or a spacer arm consisting of a saturated or unsaturated, cyclic or non-cyclic hydrocarbon-based chain optionally interrupted with one or more oxygen and/or nitrogen atoms; L₂ is a saturated or unsaturated, non-cyclic hydrocarbon-based chain containing at least 4 carbon atoms, optionally interrupted with one or more oxo groups and optionally substituted with one or more -OH groups and/or one or more chlorine atoms; W is a reactive group capable of reacting:
- either with the amine functions of the diamine, the molar ratio of the acid functions of the diacid to the reactive functions of the modifier compound ranging from 1 to 8, it being understood that the number of moles of amine functions of the diamine is equal to the sum of the number of moles of the abovementioned acid and reactive functions,
- or with the acid functions of the diacid, the molar ratio of the amine functions of the diamine to the reactive functions of the modifier compound ranging from 1 to 8, it being understood that the number of moles of acid functions of the diacid is equal to the sum of the number of moles of the abovementioned amine and reactive functions.

2. Process according to Claim 1, **characterized in that** the dicarboxylic acid is an aromatic diacid, or a linear and saturated dicarboxylic acid, containing from 4 to 22 carbon atoms.

3. Process according to Claim 2, **characterized in that** the dicarboxylic acid is terephthalic acid.

4. Process according to Claim 2, **characterized in that** the dicarboxylic acid is adipic acid.

5. Process according to any one of the preceding claims, **characterized in that** the diamine is chosen from polyetheramines comprising a linear or branched polyether chain, each end of which bears a primary amine group, diamines and polyamines which are branched, the chain of which comprises a sulfur atom, and diamine dimers derived from plant fatty acids which are optionally partially or totally hydrogenated.

6. Process according to Claim 5, **characterized in that** the diamine is chosen from diamine dimers derived from plant fatty acids which are optionally partially or total hydrogenated.

7. Process according to any one of the preceding claims, **characterized in that** the associative group A is chosen from imidazolidonyl, triazolyl, triazinyl, bisureyl and ureidopyrimidyl groups, preferably the imidazolidonyl group.

8. Process according to any one of the preceding claims, **characterized in that** the modifier compound corresponds to Formula (IV): 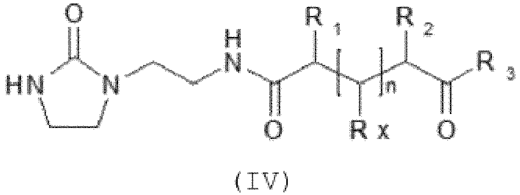 wherein: R₁, R₂ and each of the Rx groups independently denote a hydrogen atom, an -OH group or a -CH₃ group; n ranges from 2 to 12; and R₃ denotes an -OH group, an -OCH₃ group or a chlorine atom.

9. Process according to Claim 8, **characterized in that** the modifier compound corresponds to Formula (V) below: 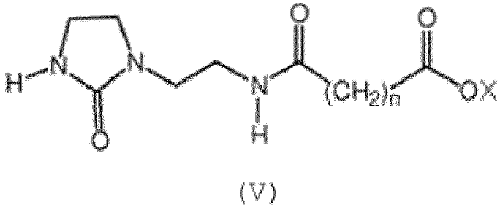 wherein n is between 4 and 14 and X=H or CH₃.
